# EUROPÄISCHE PATENTSCHRIFT

(11) **EP 1 135 182 B1**
(45) Veröffentlichungstag und Bekanntmachung des Hinweises auf die Patenterteilung: **03.11.2004**
(21) Anmeldenummer: 99958060.8
(22) Anmeldetag: 17.11.1999
(51) Int. Cl.: A61M 1/10, F04D 29/24, F04D 29/44

(54) **LAGERLOSE BLUTPUMPE**
BLOOD PUMP WITHOUT BEARING
POMPE A SANG SANS PALIER

(30) Priorität: 02.12.1998 DE 29821565 U
(43) Veröffentlichungstag der Anmeldung: 26.09.2001
(73) Patentinhaber: Impella CardioSystems AG, 52074 Aachen (DE)
(72) Erfinder: SIESS, Thorsten, D-52146 Würselen (DE)
(74) Vertreter: Selting, Günther, Dipl.-Ing.
(86) Internationale Anmeldenummer: PCT/EP1999/008835
(87) Internationale Veröffentlichungsnummer: WO 2000/032256

(56) Entgegenhaltungen:
- WO-A-98/04834
- US-A- 5 290 236
- US-A- 5 385 581

## Beschreibung

Die Erfindung betrifft eine lagerlose Blutpumpe nach dem Rotationspumpenprinzip zur vorübergehenden oder langfristigen Blutförderung.

Für die vorübergehende kurzzeitige Blutförderung werden extrakorporale Blutpumpen eingesetzt, die ein drehend angetriebenes Flügelrad aufweisen. Das Flügelrad ist in dem Pumpengehäuse auf Lagern gelagert. Beispiele solcher Blutpumpen sind in EP 0 451 376 B1 und DE 43 21 260 C1 beschrieben. Der Antrieb des Flügelrades erfolgt über eine Magnetkupplung durch einen außerhalb des Pumpengehäuses rotierenden Rotor. Die das Flügelrad stützenden Lager sind bei einer Blutpumpe problematisch, weil an den Lagern Thrombenbildung auftreten kann. Weiterhin besteht die Gefahr, daß der Abrieb von Lagern das Blut verunreinigt. Auch Dichtungen, die Lager gegen das Eindringen von Blut schützen sollen, haben sich speziell für den mittel- bis langfristigen Einsatz (Tage bis Jahre) als ungeeignet erwiesen. Blutpumpen mit mechanischen Lagerungen des Flügelrades sind aus den genannten Gründen für den langfristigen Einsatz nicht geeignet. Pumpsysteme mit Magnetlagern (US 5 385 581 A, DE 196 13 388 Al), die das Flügelrad mit einer elektromagnetischen Lagereinrichtung berührungslos lagern, führen zu einem erheblichen Regelaufwand und infolge der komplexen Lagerung zu einer voluminösen Bauform, bei der aufgrund der aktiven Flügelradzentrierung in erheblichem Maße Mehrenergie zugeführt werden muß.

Dokument WO-A-9 804 834 offenbart eine Blutpumpe mit den Merkmalen des Oberbegriffs von Anspruch 1.

Der Erfindung liegt die Aufgabe zugrunde, eine Blutpumpe mit einem in einem Pumpengehäuse rotierenden Flügelrad zu schaffen, bei der die Gefahr der Blutschädigung und Thrombenbildung minimiert ist.

Die Lösung dieser Aufgabe erfolgt erfindungsgemäß mit den im Anspruch 1 angegebenen Merkmalen.

Die erfindungsgemäße Blutpumpe ist eine lagerlose Blutpumpe, die keine mechanischen Lager aufweist. Das Flügelrad ist in dem Pumpengehäuse innerhalb eines begrenzten Spiels frei beweglich. Das Flügelrad wird von einer externen magnetischen Antriebseinrichtung gedreht und wirkt hierbei selbstzentrierend. Wenigstens eine Stirnseite der Flügel weist Tragflächen auf, welche bei Rotation das Flügelrad hydrodynamisch anheben. Die statisch wirkende Anziehungskraft der Permanentmagnete in Flügelrad und Antriebseinrichtung ist bestrebt, das Flügelrad gegen die der Antriebseinrichtung zugewandte Wand des Pumpengehäuses zu drücken. Die Tragflächen im Flügelrad bewirken bei Rotation jedoch ein Anheben des Flügelrades von der Bodenfläche, so daß das Flügelrad auf einem Blutpolster gleitet und dadurch im Abstand von der Wand gehalten wird. Hierbei wird das lagerlose Flügelrad über Permanentmagnete in Kombination mit hydrodynamisch wirkenden Antriebskräften passiv im Pumpengehäuse zentriert. Die seitliche Zentrierung des Flügelrades erfolgt ebenfalls durch die mit der Antriebseinrichtung zusammenwirkenden Magnete. Auf diese Weise gelingt es, eine lagerlose und wellenlose Blutpumpe zu schaffen, bei der das Flügelrad im Pumpengehäuse schwebend gehalten wird.

Die erfindungsgemäße lagerlose Blutpumpe bietet den Vorteil, daß infolge des Verzichts auf Lager und Gleitdichtungen die Gefahr der Thrombenbildung des Blutes und die Gefahr des Eindringens von Fremdkörpern ins Blut verringert ist. Daher kann die erfindungsgemäße Blutpumpe nicht nur als extrakorporale Blutpumpe für den Kurzzeiteinsatz benutzt werden, sondern auch als implantierbare Blutpumpe für den Langzeitbetrieb. Die Blutpumpe kann wegen der geringen zentrierbedingten Verluste mit hohem Wirkungsgrad betrieben werden, wobei die erforderliche Leistung im Bereich von 6 W unter physiologisch relevanten Betriebszuständen ist, so daß die Pumpe auch als batteriebetriebenes tragbares Gerät eine lange Betriebsdauer hat.

Das Flügelrad kann einen vom Einlaß bis zu einer Bodenwand des Pumpengehäuses gerade durchgehenden Durchlaß aufweisen. Hierdurch verfügt das Flügelrad über eine beidseitige Beschaufelung.

Vorzugsweise sind die Flügel des Flügelrades von der Umfangswand eines scheibenförmigen oder kegelförmigen Tragkörpers nach entgegengesetzten Seiten abstehend angeordnet. Das Flügelrad bildet keine Scheibe, die zusammen mit der Bodenwand des Pumpengehäuses einen engen Spalt begrenzen würde. Auch hierdurch ist die Thrombosegefahr herabgesetzt. In allen Bereichen des Pumpengehäuses wird eine Blutströmung aufrechterhalten, ohne daß die Gefahr von Totwasserbereichen besteht.

Die Flügel sind in Draufsicht im wesentlichen dreieckförmig und sie enthalten die flügelradseitigen Magnete. Durch die Dreieckform der Flügel wird erreicht, daß das Flügelvolumen mit zunehmendem Radius ansteigt, so daß die zwischen den Flügeln verfügbare Flüssigkeitsdurchtrittsfläche auf allen Radien konstant gehalten werden kann. Dadurch wird die Konizität des Pumpengehäuses, die an sich erforderlich wäre damit auf allen Umfangskreisen etwa das gleiche Volumen verfügbar ist, verringert oder eliminiert.

Die erfindungsgemäße Blutpumpe ist eine Zentrifugalpumpe, bei der der Auslaß im wesentlichen tangential zum äußeren Rand des Pumpengehäuses angeordnet ist. Da im Auslaß der größte Druck herrscht, wird eine radiale Kraft erzeugt, die bestrebt ist, das Flügelrad von dem Auslaß fortzudrücken. Um dieser dezentrierenden Kraft entgegenzuwirken, ist gemäß einer bevorzugten Weiterbildung der Erfindung an dem Pumpengehäuse ein umlaufender Ringdiffusor vorgesehen, der in einem tangentialen Auslaß mündet. Der Ringdiffusor ist ein spiralförmiger Kanal, der bewirkt, daß der im Auslaß herrschende Druck sich auf den Umfang des Pumpengehäuses verteilt und somit zentrierend auf das Flügelrad einwirkt.

Im folgenden werden unter Bezugnahme auf die Zeichnungen Ausführungsbeispiele der Erfindung näher erläutert.

Es zeigen:
- Fig. 1: einen schematischen Längsschnitt durch eine erste Ausführungsform der Blutpumpe,
- Fig. 2: eine perspektivische Ansicht des Pumpengehäuses der Blutpumpe von Fig. 1,

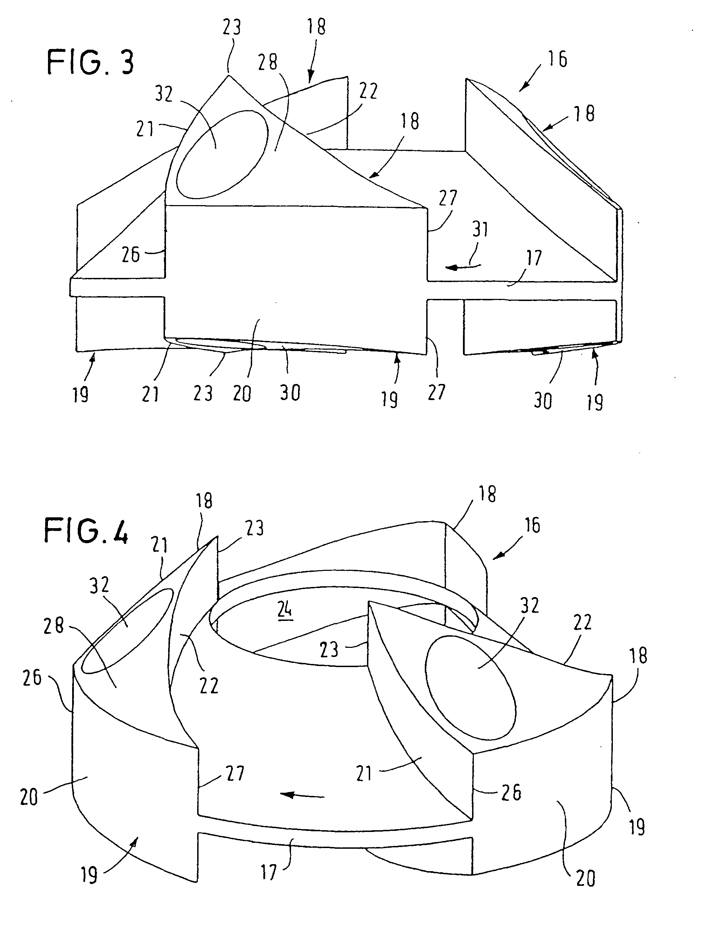

- Fig. 3: eine Ansicht des Flügelrades der Pumpe von Fig. 1,
- Fig. 4: eine andere perspektivische Ansicht des Flügelrades der Pumpe von Fig. 1,
- Fig. 5: eine zweite Ausführungsform der Blutpumpe,
- Fig. 6: eine perspektivische Ansicht des Pumpengehäuses der Pumpe von Fig. 5,
- Fig. 7: eine perspektivische Ansicht des Flügelrades der Pumpe von Fig. 5,
- Fig. 8: eine dritte Ausführungsform der Blutpumpe,
- Fig. 9: das Pumpengehäuse der Blutpumpe nach Fig. 8, und
- Fig. 10: das Flügelrad der Blutpumpe von Fig. 8.

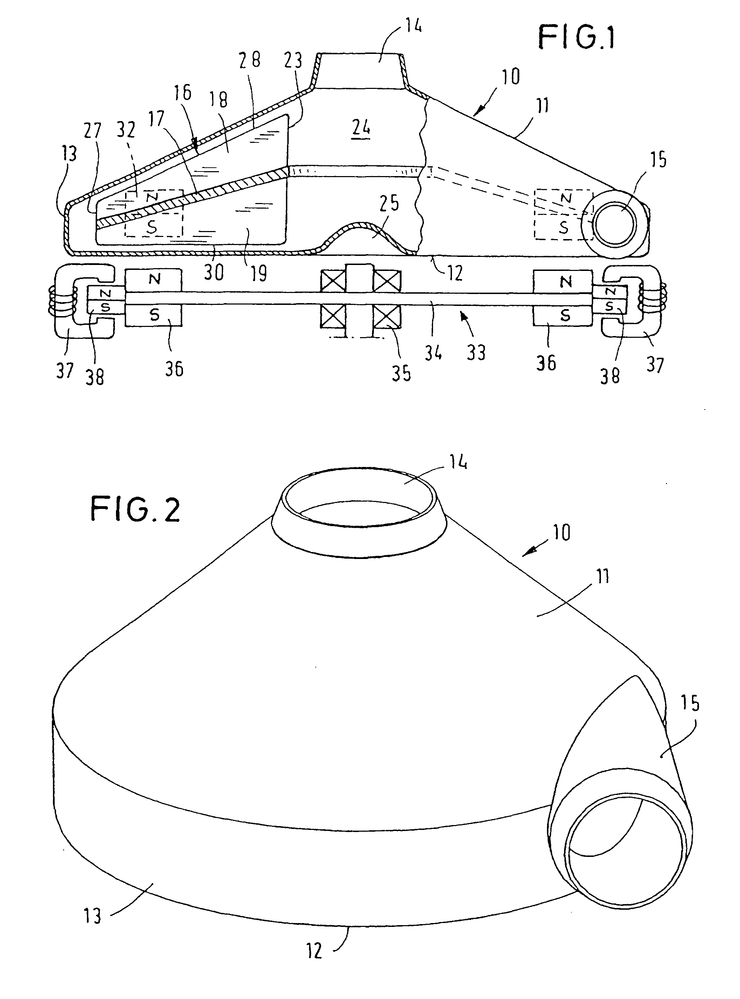

Die Blutpumpe nach Fig. 1 weist ein Pumpengehäuse 10 auf, das eine kegelstumpfförmige Umfangswand 11, eine im wesentlichen ebene Bodenwand 12 und eine zwischen Bodenwand 12 und Umfangswand 11 umlaufende Zylinderwand 13 aufweist. Das Blut wird durch den axialen Einlaß 14 dem Pumpengehäuse zugeführt und verläßt dieses durch den tangentialen Auslaß 15 am äußeren Gehäuseumfang.

In dem Pumpengehäuse 10 ist ein Flügelrad 16 drehbar untergebracht. Dieses Flügelrad weist einen kegelstumpfförmigen Tragkörper 17 auf, dessen Steigung in etwa halb so groß ist wie diejenige der Umfangswand 11. Der Tragkörper 17 besteht aus Flächenmaterial, das an allen Stellen etwa die gleiche Stärke hat. An dem Tragkörper 17 sind nach oben und unten abstehend Flügel 18,19 angeordnet, wobei die oberen Flügel 18 und die unteren Flügel 19 in Draufsicht gesehen kongruent sind, d.h. gleiche Projektionsflächen aufweisen. Die Flügel 18,19 sind in Draufsicht dreieckförmig, wobei sie eine mit dem Umfangskreis des Tragkörpers 17 zusammenfallende konvexe Umfangsfläche 20, eine in Drehrichtung vorlaufende konvexe Vorlauffläche 21 und eine konkave Innenfläche 22 aufweisen. Die konvexe Vorlauffläche 21 trifft mit der konkaven Innenfläche 22 entlang der Innenkante 23 zusammen. Der Kreis, auf dem die Innenkanten 23 der drei Flügelpaare liegen, bildet die Begrenzung eines kreisförmigen Durchlasses 24, der in axialer Verlängerung des Einlasses 14 angeordnet ist. Dies bedeutet, daß das Flügelrad 16 im Zentrum offen ist, so daß ein direkter axialer Durchlaß 24 bis zur Bodenwand 12 besteht, wobei in der Bodenwand 12 eine in den Durchlaß 24 hineinragende zentrische Erhebung 25 vorgesehen ist. Der Querschnitt des Durchlasses 24 ist mindestens so groß wie derjenige des Einlasses 14.

Bei der Drehung des Flügelrades verläuft jeweils die Innenkante 23 der Außenkante 26 derselben Vorlauffläche 21 voraus. Dies bedeutet, daß die Vorlauffläche 21 das Medium radial nach außen drückt, in dem das Medium mit Drall beaufschlagt wird. Die nachlaufende Kante 27 verläuft entlang der gleichen Bahn wie die vorlaufende Kante 26.

Die Oberseite 28 der oberen Flügel 18 bewegt sich in einer kegelstumpfförmigen Ebene, die den gleichen Kegelwinkel hat wie die Umfangsfläche 11 des Pumpengehäuses. Zwischen den Oberseiten 28 der Flügel und der kegelförmigen Umfangsfläche 11 des Pumpengehäuses besteht ein Spalt, der das für eine Axialbewegung des Flügelrades erforderliche Spiel zur Verfügung stellt.

Die Unterseiten der unteren Flügeln 19 bilden Tragflächen 30, welche bei Rotation des Flügelrades in Richtung des Pfeiles 31 das Flügelrad von der Bodenwand 12 des Pumpengehäuses abheben. Diese Tragflächen werden dadurch gebildet, daß an der Unterseite des Flügels die Unterkante der Vorlauffläche 21 einen größeren Abstand von der Bodenwand 12 hat als an dem nachlaufenden Ende, nämlich an der Kante 27. Auf diese Weise entsteht zwischen der Tragfläche 30 und der Bodenwand 12 ein Spalt, der sich zum nachlaufenden Ende hin verringert, so daß die in dem Spalt befindliche Flüssigkeit bestrebt ist, das Flügelrad anzuheben. Außerdem wird die vertikale Höhe des Spaltes über der Bodenwand von der Innenkante 23 aus nach außen größer, wodurch auch eine radiale Zentrierung des Flügelrades erfolgt. Der Neigungswinkel α, den die Tragfläche 30 in Umfangsrichtung hat, beträgt ca. 2 bis 4°.

Die in Draufsicht dreieckförmigen Flügel 18,19 enthalten jeweils einen Magneten 32 mit Nordpol N und Südpol S. Dieser Magnet erstreckt sich durch beide Flügel 18,19 hindurch.

Der Antrieb der Blutpumpe erfolgt durch eine externe magnetische Antriebseinrichtung 33, auf die das Pumpengehäuse 10 aufgesetzt wird. Diese Antriebseinrichtung enthält einen Rotor 34, der in Lagern 35 gelagert ist und an seinem Umfang Magnete 36 trägt. Die Magnete 36 ziehen jeweils einen Magnet 32 im Pumpengehäuse 10 an. Der Rotor 34 wird durch Elektromagnete 37 gedreht, welche ortsfest angeordnet sind. Jeder Elektromagnet 37 weist ein U-förmiges Joch auf, durch das ein Magnet 38, der am Umfang des Rotors 34 angeordnet ist, hindurchgeht. Die Elektromagnete 37 werden zyklisch umgepolt, so daß sie ein rotierendes Magnetfeld erzeugen, welches den Rotor 34 mitnimmt. Über die magnetisch gekoppelten Magnete 32 und 36 dreht der Rotor 34 das Flügelrad 16. Sämtliche Teile des Flügelrades 16, mit Ausnahme der Magnete 32, bestehen aus Kunststoff oder einem anderen nicht-magnetischen Material.

Die Art der Magnetanordnung von Rotormagnet 32 am Antriebsmagnet 36 führt zu einer radialen Zentrierung des Flügelrads 16. Hierdurch werden 2 kartesische Achsen und 3 Drehachsen definiert. Der letzte verbleibende Freiheitsgrad in Richtung der Magnetanziehung wird durch den in Umfangsrichtung verlaufenden konvergenten Spalt der Tragfläche 30 zur Bodenwand 12 fixiert. Hierdurch hebt das Flügelrad bei Rotation entgegen der Magnetanziehung von der Bodenwand 12 ab. Ist eine ausreichende Umfangsgeschwindigkeit des Flügelrads erreicht, so bildet sich im konvergenten Spalt ein tragfähiger Blutfilm und das Flügelrad schwebt ohne Mischreibung im Pumpengehäuse.

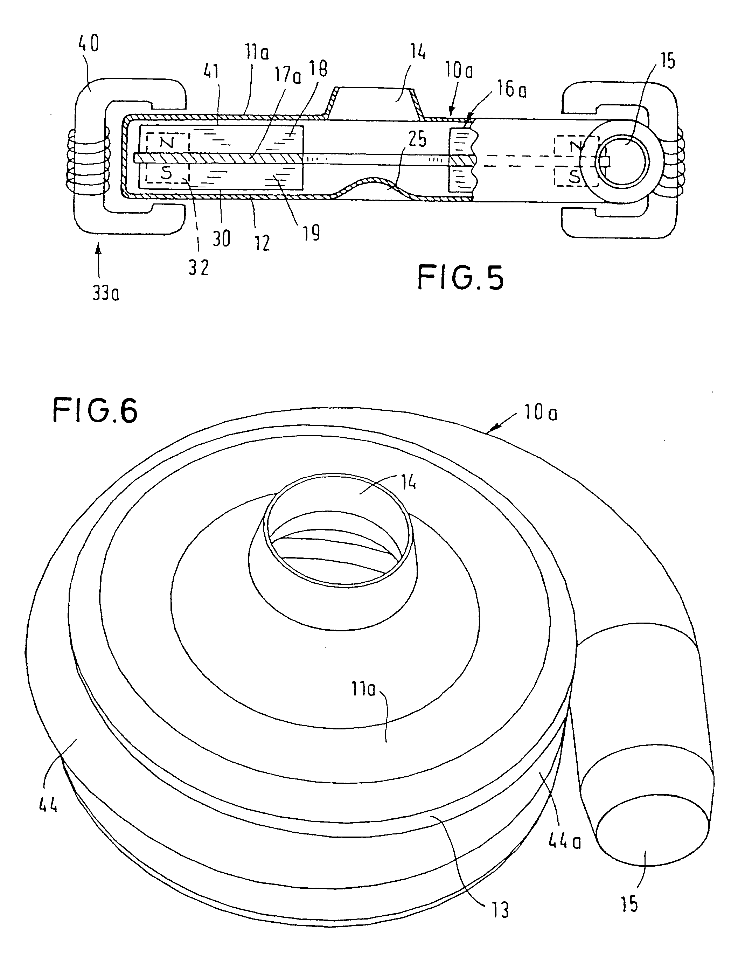

Bei dem Ausführungsbeispiel der Fig. 5 hat das Gehäuse 10a eine ebene Bodenwand 12 und eine hierzu im wesentlichen parallele ebene Oberwand 11a. Der Tragkörper 17a, von dem die Flügel 18,19 nach oben und unten abstehen, besteht hier aus einer ebenen Scheibe.

Die externe Antriebseinrichtung 33a weist gemäß Fig. 5 Elektromagnete 40 auf, die um den Umfang des Pumpengehäuses 10a herum verteilt angeordnet sind und von denen ein umlaufendes Magnetfeld erzeugt wird. Die Joche der Elektromagnete 40 wirken unmittelbar auf die Magnete 32 des Flügelrades 16a ein. Auch hier erfolgt durch die Magnete nicht nur der Drehantrieb des Flügelrades sondern auch dessen radiale Zentrierung. Zur axialen Zentrierung des Flügelrades sind die Flügel an ihrer Unterseite mit einer schrägen Tragfläche 30 und auf ihrer Oberseite mit einer ebenfalls schrägen Tragfläche 41 versehen, welche mit der Oberwand 11a des Pumpengehäuses einen konvergenten Zentrierspalt bildet.

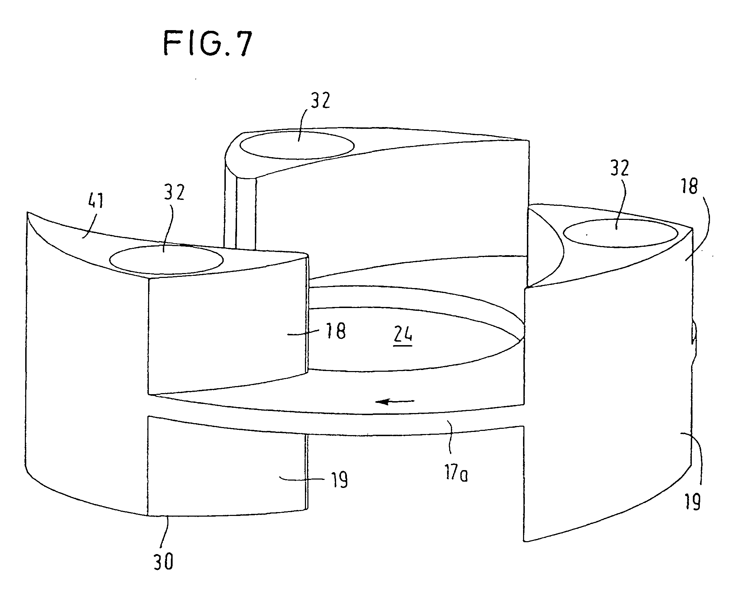

Die Flügel 18,19 haben die in Fig. 7 dargestellte Flügelform, die von derjenigen des ersten Ausführungsbeispiels aufgrund der in Drehrichtung nach vorne gekrümmten Schaufeln abweicht. In allen Fällen reichen die Flügel bis zum Durchlaß 24 und die Flügelbreite (in Umfangsrichtung) nimmt vom Durchlaß 24 aus nach außen zu, so daß jeder Flügel an dem Rand des Tragkörpers 17 bzw. 17a die größte Breite hat.

Das Pumpengehäuse 10a hat gemäß Fig. 6 generell die Form eines flachen Zylinders mit ebener Oberwand 11a und zylindrischer Umfangswand 13. Da sich bei Rotation des Flügelrades 16a im Auslaß 15 der größte Druck aufbaut, kann es geschehen, daß dieser Druck das Flügelrad gegen die dem Auslaß gegenüberliegende Seite des Pumpengehäuses drückt. Um diese Druckkraft zu kompensieren, erstreckt sich um den Umfang des Pumpengehäuses ein Ringdiffusor 44, der das Pumpengehäuse umfangsmäßig voll umschließt und aus einer spiralförmigen Ausbauchung besteht, deren Querschnitt sich vom Anfangsende 44a bis zum Auslaß 15 kontinuierlich vergrößert.

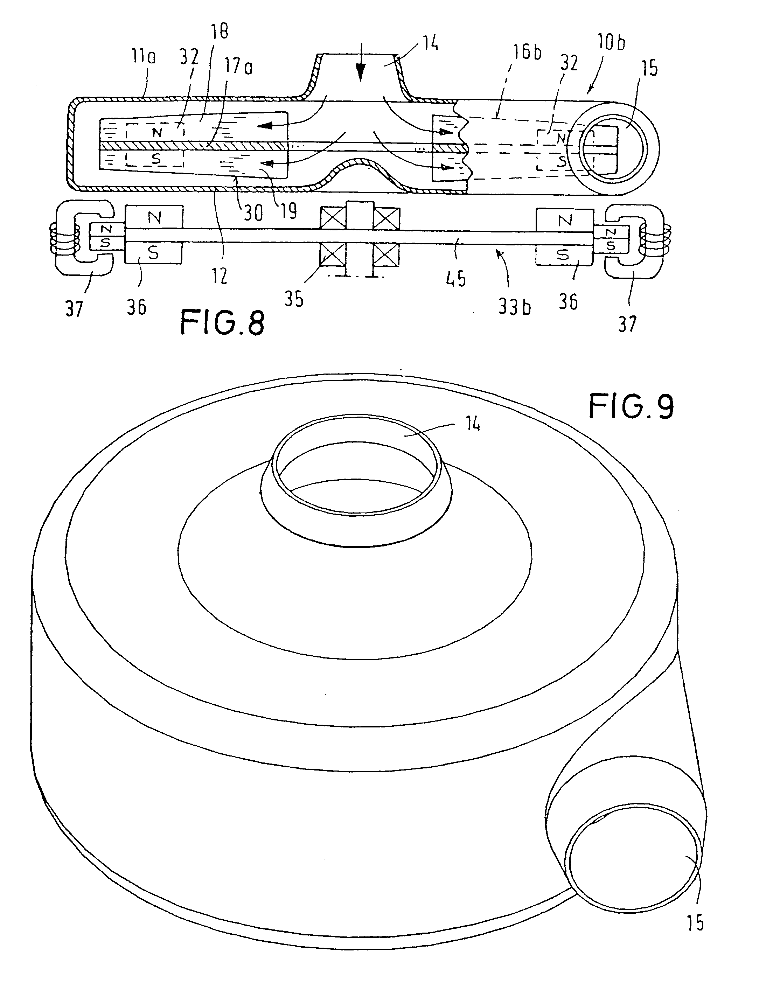

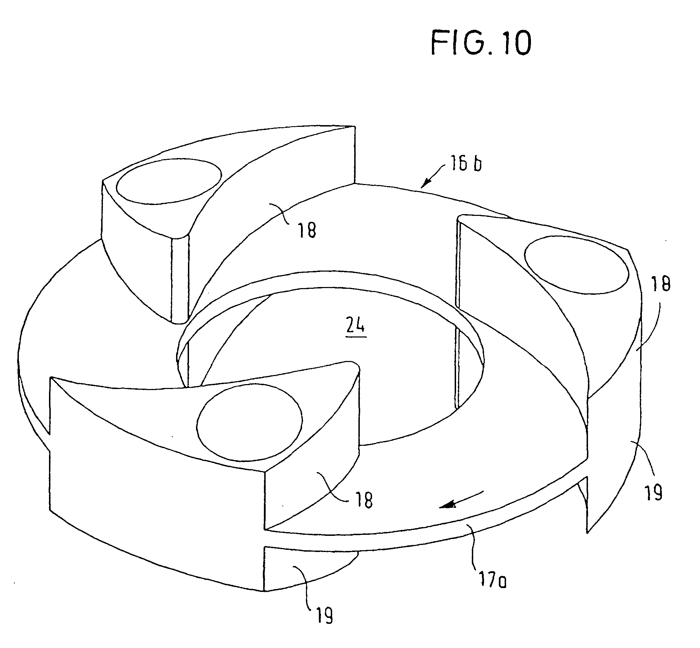

Das Ausführungsbeispiel der Fign. 8 bis 10 entspricht weitgehend demjenigen der Fign. 5 bis 7. Das Pumpengehäuse 10b hat im wesentlichen die Form eines flachen Zylinders mit ebener Oberseite 11a und ebener Bodenwand 12. Die Unterseite der unteren Flügel 19 bildet eine hydrodynamische Tragfläche 30, die ebenso wie bei den vorherigen Ausführungsbeispielen zur Vorlaufkante hin ansteigt. Außerdem steigt die Tragfläche 30 gemäß Fig. 8 nach außen hin an.

Die Antriebseinrichtung 33b besteht aus einem Scheibenläufermotor 45, der in Lagern 35 gelagert ist und Magnete 36 trägt, welche mit den Magneten 32 des Flügelrades 16b zusammenwirken.

## Patentansprüche

1. Blutpumpe mit einem Pumpengehäuse (10), das einen axialen Einlaß (14) und einen an seinem Umfang angeordneten Auslaß (15) aufweist, und einem in dem Pumpengerhäuse (10) drehbar angeordneten Flügelrad (16), welches Magnete (32) zum Zusammenwirken mit einer externen magnetischen Antriebseinrichtung (33) aufweist, wobei das Flügelrad (16) in dem Pumpengehäuse (10) innerhalb eines begrenzten Spiels frei beweglich ist und die Flügel (19) an der der Antriebseinrichtung (33) zugewandten Unterseite das Flügelrad (16) hydrodynamisch anhebende Tragflächen (30) aufweisen,
**dadurch gekennzeichnet,**
**dass** das Flügelrad (16) lagerlos positioniert ist und in Verlängerung des axialen Einlasses (14) des Pumpengehäuses (10) einen Durchlass (24) aufweist.

2. Blutpumpe nach Anspruch 1, **dadurch gekennzeichnet, daß** das Flügelrad (16) wellenlos ist und einen vom Einlaß (14) bis zu einer Bodenwand (12) gerade durchgehenden Durchlaß (24) aufweist.

3. Blutpumpe nach Anspruch 1 oder 2, **dadurch gekennzeichnet, daß** die Flügel (16,19) von der Flächenwand eines scheibenförmigen oder kegelstumpfförmigen Tragkörpers (17) nach entgegengesetzten Seiten abstehend angeordnet sind.

4. Blutpumpe nach einem der Ansprüche 1 bis 3, **dadurch gekennzeichnet, daß** die Flügel (18,19) in Draufsicht im wesentlichen dreieckförmig sind, wobei die Flügelbreite mit zunehmendem Radius ansteigt.

5. Blutpumpe nach einem der Ansprüche 1 bis 4, **dadurch gekennzeichnet, daß** die Flügel (18,19) die Magnete (32) enthalten.

6. Blutpumpe nach Anspruch 4, **dadurch gekennzeichnet, daß** die Flügel (18,19) eine konvexe Außenfläche (20) haben, die auf dem kreisförmigen Rand eines Trägerkörpers (17) liegt.

7. Blutpumpe nach Anspruch 6, **dadurch gekennzeichnet, daß** die Tragflächen (30) der Flügel (19) zu einer nachlaufenden Kante (27) der Dreleckform hin abfallen.

8. Blutpumpe nach einen der Ansprüche 1 bis 7, **dadurch gekennzeichnet, daß** an dem Pumpengehäuse (10a) ein umlaufender Ringdiffusor (44) vorgesehen ist, der in den tangentialen Auslaß (15) mündet.

## Claims

1. Blood pump without bearing, having a pump casing (10) comprising an axial inlet (14) and an outlet (15) disposed on its circumference, and having an impeller (16) rotatably arranged in said pump casing (10), said impeller (16) being provided with magnets (32) adapted to cooperate with an external magnetic driving means (33), wherein the impeller (16) is freely movable within a limited clearance in the pump casing (10), and the blades (19) on the lower side facing the driving means (33) comprise supporting surfaces (30) hydrodynamically lifting the impeller (16),
**characterized in that**
the impeller (16) is positioned without any bearing involved and comprises a passage (24) in extension of the axial inlet (14) of the pump casing (10).

2. Blood pump according to claim 1, **characterized in that** the impeller (16) has no shaft and comprises a straight continuous passage (24) extending from the inlet (14) to a bottom wall (12).

3. Blood pump according to claim 1 or 2, **characterized in that** the blades (18,19) are arranged such that they protrude to opposite sides from the surface wall of a disk-shaped or truncated supporting body (17).

4. Blood pump according to one of claims 1 to 3, **characterized in that**, as seen from the top, the blades (18,19) are of essentially triangular configuration, wherein the blade width increases with increasing radius.

5. Blood pump according to one of claims 1 to 4, **characterized in that** the blades (18,19) comprise the magnets (32).

6. Blood pump according to claim 4, **characterized in that** the blades (18, 19) have a convex outer surface (20) on the circular border of a supporting body (17).

7. Blood pump according to claim 6, **characterized in that** the supporting surfaces (30) of the blades (19) slope towards a trailing edge (27) of the triangular shape.

8. Blood pump according to one of claims 1 to 7, **characterized in that** a peripheral ring diffusor (44) is provided on the pump casing (10a), the ring diffusor (44) ending in the tangential outlet (15).

## Revendications

1. Pompe à sang comprenant un boîtier de pompe (10) qui présente une admission axiale (14) et une évacuation (15) disposée sur sa périphérie, et comprenant une roue à ailettes (16) disposée en rotation dans le boîtier de pompe (10) qui présente des aimants (32) pour coopérer avec un dispositif d'entraînement (33) magnétique externe, la roue à ailettes (16) étant mobile librement dans le boîtier de pompe (10) à l'intérieur d'un jeu limité et les ailettes (19) présentant des surfaces d'appui (30) à levage hydrodynamique sur la face inférieure tournée vers le dispositif d'entraînement (33) de la roue à ailettes (16), **caractérisée en ce que** la roue à ailettes (16) est positionnée sans palier et présente un passage (24) dans le prolongement de l'admission axiale (14) du boîtier de pompe (10).

2. Pompe à sang selon la revendication 1, **caractérisée en ce que** la roue à ailettes (16) est sans arbre et présente un passage (24) traversant de manière rectiligne à partir de l'admission (14) et jusqu'à une paroi de fond (12).

3. Pompe à sang selon la revendication 1 ou 2, **caractérisée en ce que** les ailettes (18, 19) sont disposées en saillie à partir de la paroi de surface d'un corps support (17) en forme de disque ou de cône tronqué vers des côtés opposés.

4. Pompe à sang selon l'une des revendications 1 à 3, **caractérisée en ce que** les ailettes (18, 19) ont une forme essentiellement triangulaire en vue de dessus, la largeur des ailettes augmentant avec un rayon croissant.

5. Pompe à sang selon l'une des revendications 1 à 4, **caractérisée en ce que** les ailettes (18, 19) contiennent les aimants (32).

6. Pompe à sang selon la revendication 4, **caractérisée en ce que** les ailettes (18, 19) ont une surface externe (20) convexe qui se trouve sur le bord circulaire d'un corps support (17).

7. Pompe à sang selon la revendication 6, **caractérisée en ce que** les surfaces d'appui (30) des ailettes (19) descendent jusqu'à une arête (27) arrière de la forme triangulaire.

8. Pompe à sang selon l'une des revendications 1 à 7, **caractérisée en ce qu'**un diffuseur annulaire (44) périphérique est prévu sur le boîtier de pompe (10a) et débouche dans l'évacuation tangentielle (15).
